(19) Europäisches Patentamt
European Patent Office
Office européen des brevets

(11) Veröffentlichungsnummer: **0 467 407 B1**

# (12) EUROPÄISCHE PATENTSCHRIFT

(45) Veröffentlichungstag der Patentschrift: **29.11.95**

(51) Int. Cl.[6]: **C07C 49/747**, C07C 50/38, C07D 311/92, A61K 31/12

(21) Anmeldenummer: **91112192.9**

(22) Anmeldetag: **19.07.91**

(54) **Pharmazeutisch brauchbare Naphthalin- und Anthracenderivate.**

(30) Priorität: **20.07.90 DE 4023159**

(43) Veröffentlichungstag der Anmeldung:
**22.01.92 Patentblatt 92/04**

(45) Bekanntmachung des Hinweises auf die Patenterteilung:
**29.11.95 Patentblatt 95/48**

(84) Benannte Vertragsstaaten:
**AT BE CH DE DK ES FR GB GR IT LI LU NL SE**

(56) Entgegenhaltungen:
**US-A- 4 283 390**

**CHEMICAL ABSTRACTS, vol. 110, 1989, Columbus, Ohio, US; abstract no. 82476m, & JP-A-62 207 213**

**CHEMICAL ABSTRACTS, vol. 107, 1987, Columbus, Ohio, US; abstract no. 230651,**

**JOURNAL OF THE AMERICAN CHEMICAL SOCIETY. Bd. 97, Nr. 17, 1975, GASTON, PA US Seiten 4985 - 4990 D.L. DREYER ET AL**

(73) Patentinhaber: **Pineyro-Lopez, Alfredo, Prof. Dr.,
Rio Mississippi No. 253 Ote.
Col. Del Valle,
Nuevo Leon (MX)**

Patentinhaber: **UNIVERSIDAD AUTONOMA DE NUEVO-LEON
Apdo. Postal 1-4469
Monterrey,
N.L., C.P. 64460 (MX)**

(72) Erfinder: **Pineyro-Lopez, Alfredo Prof. Dr.
Rio Mississippi No. 253 ote.
Col. Del Valle, Nuevo Leon (MX)**

(74) Vertreter: **Kinzebach, Werner, Dr. et al
Patentanwälte
Reitstötter, Kinzebach und Partner
Postfach 86 06 49
D-81633 München (DE)**

EP 0 467 407 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, vol. 110, 1989, Columbus, Ohio, US; abstract no. 228622e

CHEMICAL ABSTRACTS, vol. 80, 1974, Columbus, Ohio, US; abstract no. 12463t

CHEMICAL ABSTRACTS, vol. 78, 1973, Columbus, Ohio, US; abstract no. 4001j,

CHEMICAL ABSTRACTS; vol. 80, 1974, Columbus, Ohio, US; abstract no. 12461r,

## Beschreibung

Karwinskia humboldtiana ist ein Strauch aus der Familie der Rhamnaceen, der in den Halbwüsten von Nord- und Zentralmexiko und im Südwesten der USA häufig vorkommt und z.B. in Hagers Handbuch der pharmazeutischen Praxis, 4. Ausgabe, V. Band, S. 397, beschrieben ist. Die Pflanze ist dadurch aufgefallen, daß beim Genuß von Pflanzenteilen Lähmungserscheinungen beobachtet worden sind, deren Symptome ähnlich denjenigen des Guillain-Barré-Syndroms, der Poliomyelitis und anderer peripherer Polyneuropathien sind.

Weiter wurde von extraneuronalen Schäden bei Ziegen und Schafen berichtet.

Untersucht wurden bisher vor allem die Früchte. Dreyer et al. in J. Am Chem. Soc. 1975, 97, 4986 konnte aus dem Endokarp dieser Früchte vier dimere Anthracenverbindungen isolieren und auftrennen. Diese Verbindungen erhielten entsprechend ihrem Molekulargewicht die Kurzbezeichnungen T-496, T-514, T-516 und T-544.

Überraschenderweise wurde nun gefunden, daß bestimmte Anthracen-Verbindungen eine selektive cytostatische und eine cytotoxische sowie antivirale Wirkung haben. Besonders vorteilhaft war die Wirkung auf Tumorzellen.

Die vorliegende Erfindung betrifft daher pharmazeutische Mittel, die Verbindungen der Formel 1:

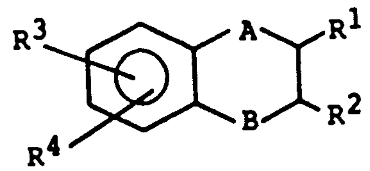

1

worin

A für C = O oder C-OH steht und

B für CH-$R^5$ oder C-$R^5$ steht, wobei der die Gruppen A und B aufweisende Ring aromatisch ist, wenn A für C-OH und B für C-$R^5$ stehen,

$R^1$ und $R^2$, die gleich oder verschieden sein können, für eine $C_1$-$C_4$-Acylgruppe, $C_1$-$C_4$-Alkylgruppe, $C_1$-$C_4$-Alkoxygruppe steht oder $R^1$ und $R^2$ zusammen mit den Kohlenstoffatomen, an die sie gebunden sind, für einen Phenyl-, Cyclohexanon- oder Tetrahydropyranring stehen, der durch mindestens einen Rest substituiert sein kann, der ausgewählt ist unter einer $C_1$-$C_4$-Alkylgruppe, $C_1$-$C_4$-Alkoxygruppe, Hydroxygruppe, und $C_1$-$C_4$-Acyloxygruppe,

$R^3$ für eine $C_1$-$C_4$-Alkylgruppe, $C_1$-$C_4$-Alkoxygruppe, $C_1$-$C_4$-Acyloxygruppe oder Hydroxygruppe steht, und

einer der Reste $R^4$ und $R^5$ für ein Wasserstoffatom und der andere für eine Gruppe der Formel:

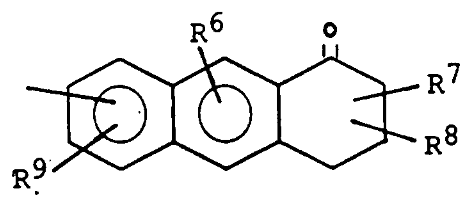

steht, worin $R^6$, $R^7$, $R^8$ und $R^9$ eine $C_1$-$C_4$-Alkylgruppe, $C_1$-$C_4$-Alkoxygruppe, $C_1$-$C_4$ Acyloxygruppe oder Hydroxygruppe bedeuten,

oder deren tautomere Formen, Stellungsisomere und optische Isomere enthalten.

Eine bevorzugte Ausführungsform sind die Verbindungen der Formel:

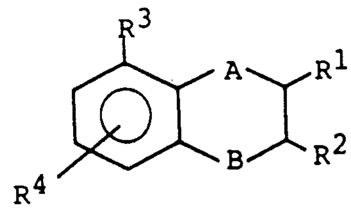

worin A, B und $R^1$-$R^4$ die oben angegebenen Bedeutungen besitzen.

Vorzugsweise ist der Rest $R^4$ in ortho-Stellung zum Rest $R^3$ an den Phenylring gebunden.

Weitere bevorzugte Ausführungsformen sind die Verbindungen der Formeln 1.1, 1.2 und 1.3:

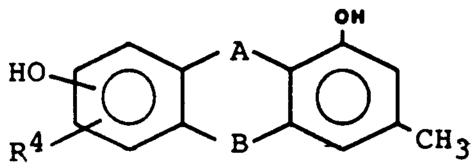

1.1

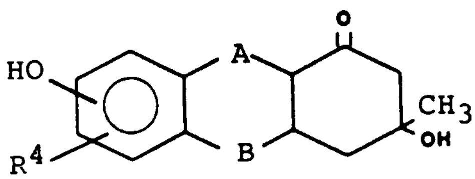

1.2

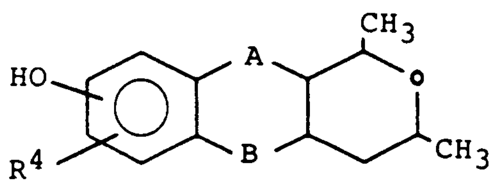

1.3

Eine weitere bevorzugte Ausführungsform sind die Verbindungen der Formel 1, worin $R^1$ für eine $C_1$-$C_4$-Acylgruppe, insbesondere eine Acetylgruppe, und $R^2$ für eine $C_1$-$C_4$-Alkylgruppe,insbesondere eine Methylgruppe, stehen.

In den oben genannten Formeln steht einer der Reste $R^4$ und $R^5$ und insbesondere der Rest $R^5$ für eine Gruppe der Formel:

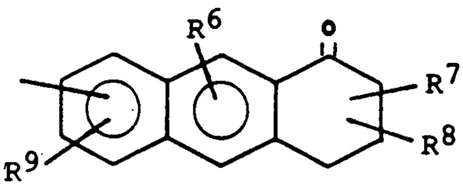

Vorzugsweise stehen $R^6$, $R^9$ und einer der Reste $R^7$ und $R^8$ für eine Hydroxygruppe und der andere der Reste $R^7$ und $R^8$ für eine $C_1$-$C_4$-Alkylgruppe, insbesondere eine Methylgruppe.

Besonders bevorzugt steht einer der Reste $R^4$ und $R^5$ für eine Gruppe der Formel:

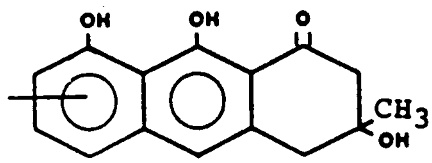

die insbesondere über ihre 2-Stollung an das Grundgerüst gebunden ist.

Vorzugsweise steht der Rest $R^3$ in den oben genannten Formeln für eine Hydroxygruppe.

Besonders bevorzugte Verbindungen sind:

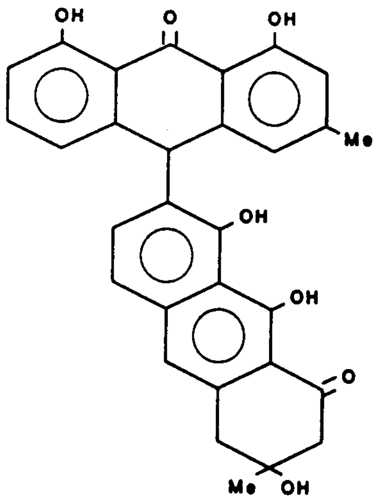

(I)

(T-496)

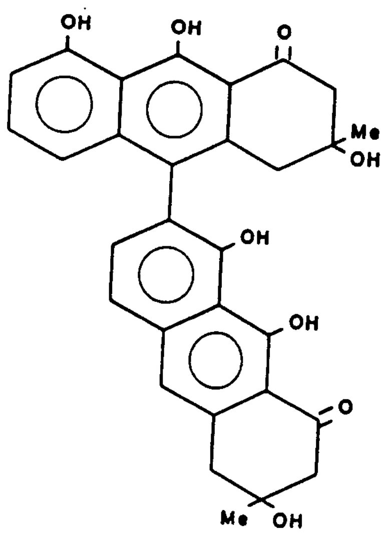

(II)

(T-514)

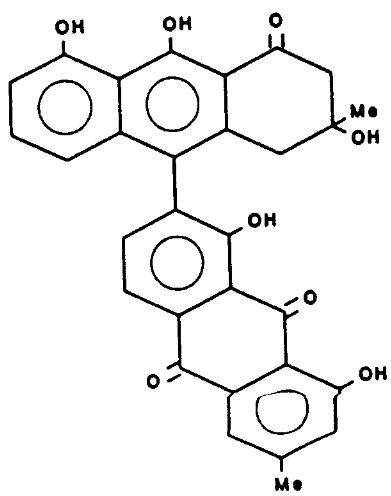

(III)

(T-510)

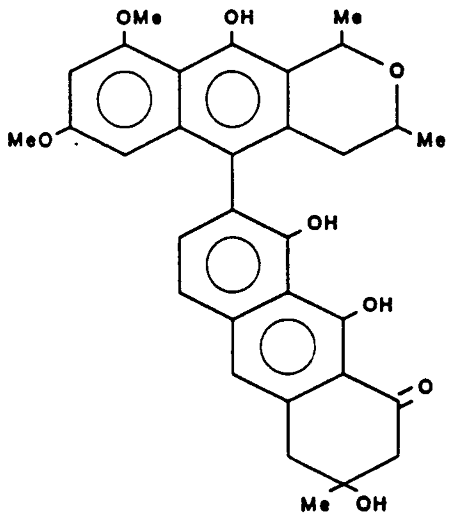

(IV)

(T-544)

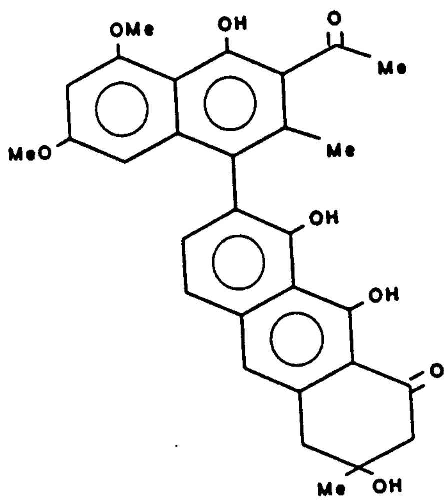

(V)

(T-516)

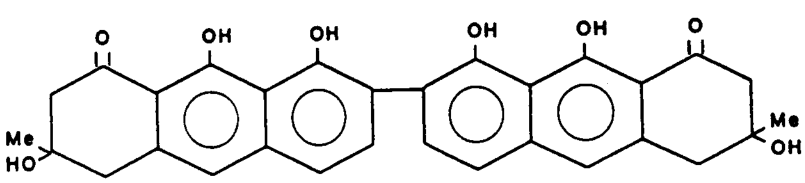

(VI)

(T-ISO514)

Im folgenden werden diese Verbindungen mit der angegebenen Kurzbezeichnung, die auf das Molekulargewicht Bezug nimmt, bezeichnet.

Das Verfahren zur Herstellung der erfindungsgemäßen Verbindungen besteht darin, daß man diese aus den Früchten der Gattung Karwinskia (oder anderer die erfindungsgemäßen Verbindungen enthaltenden Pflanzen) isoliert. Zu diesem Zweck werden die Früchte in üblicher Weise getrocknet und zerkleinert. Anschließend führt man eine Entfettung mit einem unpolaren organischen Lösungsmittel, beispielsweise Pentan, Hexan, Petrolether etc. Durch. Die entfetteten Früchte werden dann mit einem Lösungsmittel mittlerer Polarität extrahiert. Geeignete Lösungsmittel sind chlorierte aliphatische Kohlenwasserstofe, wie Chloroform, Methylenchlorid, Ethylendichlorid etc. Die Extraktion führt man in üblicher Weise durch, wobei man etwa die 3- bis 10fache Menge an Lösungsmittel, bezogen auf die zu extrahierenden Früchte (V/G), verwendet. Zweckmäßigerwise wird vor der Weiterverarbeitung der Extrakt eingeengt, beispielsweise auf ein Zehntel bis ein Zwanzigstel des ursprünglichen Volumens. Gegebenenfalls kann das Lösungsmittel auch vollständig entfernt und der Rückstand wie unten beschrieben, aufgearbeitet werden.

Aus dem eingeengten Extrakt wird durch Zugabe eines unpolaren organischen Lösungsmittels, beispielsweise Pentan, Hexan, Petrolether etc., ein Produkt ausgefällt, das eine (oder mehrere) der erfindungsgemäßen Verbindungen enthält. Dieses wird in üblicher Weise gewonnen, beispielsweise mittels Dünnschicht- oder Säulenchromatographie oder mittels fraktionierter Kristallisation.

Die auf diese Weise erhaltenen Verbindungen können durch übliche chemische Umsetzungen, wie Veresterungen, Veretherungen, Ester- und Etherhydrolysen, Substitutionsreaktionen am aromatischen Kern, Oxidationen und Reduktionen in andere erfindungsgemäße Verbindungen umgewandelt werden.

Die Verbindungen T-514, 496, 544 und 516 lassen sich aus den Früchten von Karwinskia humboldtiana beispielsweise nach dem in J. Am. Chem. Soc. 97 , 4986 (1975) beschriebenen Verfahren isolieren. Ein weiteres Verfahren zur Gewinnung von T-514 ist in Toxicon 25, Nr. 5, 565-568 (1987) beschrieben.

Pharmakologische Untersuchungen haben gezeigt, daß die cytostatische und cytotoxische Wirkung der erfindungsgemäßen Verbindungen sehr selektiv ist, insbesondere gegenüber Leber, Lunge und Kolongewebe. Die erfindungsgemäßen Verbindungen sind in der Lage, zwischen gutartigen und bösartigen Zelltypen zu unterscheiden. Sie sind daher zur Tumorbehandlung, insbesondere zur Behandlung von Leber-, Lungen- und Kolonkarzinomen, brauchbar. Außerdem hat sich gezeigt, daß die erfindungsgemäßen Verbindungen antivirale Wirkung besitzen und daher zur Behandlung von Viruserkrankungen, z.B.Herpes simplex I, II, III, brauchbar sind.

Die erfindungsgemäßen Verbindungen können entweder einzeln als therapeutische Wirkstoffe oder zusammen mit anderen therapeutischen Wirkstoffen verabreicht werden. Sie können als solche verabreicht werden, im allgemeinen werden sie jedoch in Form pharmazeutischer Mittel verabreicht, das heißt als Mischung der Wirkstoffe mit geeigneten pharmazeutischen Trägern und/oder Hilfsstoffen. Die Verbindungen oder Mittel können oral oder parenteral, vorzugsweise in parenteralen Darreichungsformen, verabreicht werden. Zu diesem Zweck kommen die Wirkstoffe in üblichen pharmazeutischen Trägern zur Anwendung, beispielsweise in Form von Infusionslösungen.

Die Herstellung der pharmazeutischen Mittel erfolgt in üblicher Weise, indem man den Wirkstoff einem pharmazeutischen Träger und/oder Hilfsstoff einverleibt.

Zur therapeutischen Behandlung können die erfindungsgemäßen Verbindungen Säugern (Mensch und Tier) in Dosen von etwa 0,01 bis etwa 2 mg, vorzugsweise etwa 0.01 mg bis etwa 1 mg und insbesondere etwa 0.01 mg bis etwa 0.5 mg oder etwa 0.01 mg bis etwa 0.1 mg pro kg Körpergewicht pro Tag verabreicht werden. Sie können in einer einzelnen Dosis oder in mehreren aufgeteilten Dosen verabreicht werden. Der genannte Dosisbereich ist nur beispielhaft. Es ist klar, daß der Arzt die am besten geeignete Dosis bestimmt, wobei solche Faktoren wie Alter, Gewicht, die zu behandelnde Krankheit, die Schwere und der Situs der Krankheit zu berücksichtigen sind.

Die folgenden Beispiele erläutern die Erfindung.

Beispiel 1

Gewinnung von 3,3',8,8',9,9'-Hexahydroxy-3,3,4,4-tetrahydro-(7,10')-bisanthracen-1,1'-(2H,2H')-dion (T-514) der Formel

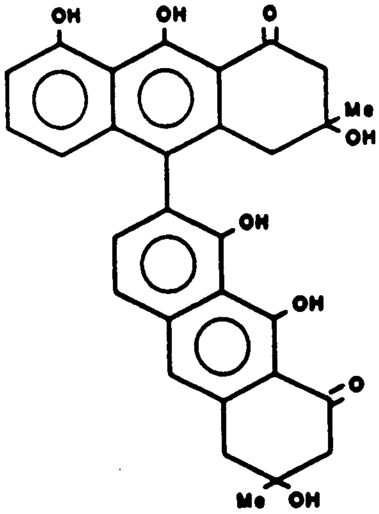

(T-514)

500 g getrocknete, zerkleinerte Früchte der Karwinskia humboldtiana werden nacheinander mit 3 l Petroläther (Siedebereich ungefähr 60 bis 70 °C) und 3 l Chloroform extrahiert. Der Chloroformextrakt wird auf 100 ml konzentriert und das Produkt durch 2-malige Zugabe von 300 ml n-Hexan gefällt. Das erhaltene gelbe Pulver wird an Silikagel G (Schichtdicke 0,5 mm) mit Benzol : Aceton(2 : 1) als Laufmittel fraktioniert. Die Fraktion mit dem niedrigsten $R_f$-Wert ist die Verbindung T-514.Diese Fraktion wird von der Chromatographieplatte abgekratzt und mit Aceton zurückeluiert. Das Produkt wird weiter an acetyliertem Polyamid (0,5 mm Schichtdicke) mit Methanol : $H_2O$(2 : 1)als Laufmittel gereinigt. Nach Umkristallisation aus Benzol-Hexan erhält man reines T-514 (50 mg).
Schmelzpunkt: 178 - 180 °C
$^1$H-NMR und $^{13}$C-NMR-Spektren sind in Tabelle 1, Beispiel 2 zusammengestellt.
$LD_{50}$ (nach intraperitonealer Verabreichung an der Maus): 6,52 ∓ 0,86 mg/kg.

Beispiel 2

Gewinnung von T-514' (optisches Isomer zu T-514)

500 g getrocknete, gemahlene Früchte von Karwinskia parvifolia wurden nacheinander mit jeweils 3 l Petroläther, Chloroform und Methanol extrahiert. Die Extraktionen erfolgten jeweils 3 Tage bei Raumtemperatur. Der Chloroformextrakt wurde nach Abdampfen des Lösungsmittels an Kieselgel mit Benzol : Aceton(3 : 1), 0,1 % Essigsäure, in drei Fraktionen aufgetrennt. Jede Fraktion wurde weiter durch Chromatographie an Kieselgel und acetyliertem Polyamid gereinigt. Aus der 1. Fraktion wurde eine als T-496 bezeichnete Verbindung, aus der 3. Fraktion die Verbindung T-514 und aus der 2. Fraktion die gewünschte Verbindung T 514' gewonnen. Umkristallisation von T-514' aus Benzol-Hexan liefert ein gelbes Pulver (450 mg) mit einem Schmelzpunkt von 169 bis 171 °C.
UV (MeOH) $_{max}$ 220 (4,83), 266 (4,97), 408 (4,38);
IR: 3360, 1625, 1350, 1250;
EIMS: m/z (rel. int.) $M^+$514 (30), 478 (80), 240 (10), 43 (100)
Die NMR-Spektren von T-514 und T-514' sind in den nachfolgenden Tabellen 1 und 2 zusammengestellt.

Tabelle 1

$^1$H NMR von T-514 und T-514'

| H | T 514 | T 514' | H | T 514 | T 514' |
|---|---|---|---|---|---|
| 2 | 2,87 | 3,00 | 2' | 2,85 | 2,95 |
| 3-Me | 1,45 | 1,3 | 3'-Me | 1,31 | 1,15 |
| 4 | 3,13 | 3,1(17,5)<br>3,0(17,5) | 4' | 2,91(16,7)<br>2,70(16,7) | 2,90(17,5)<br>2,70(17,5) |
| 5 | 7,35(8,0) | 7,39(8,0) | 5' | 6,7 (8,3) | 6,6(8,25) |
| 6 | 7,32/8,0) | 7,34(8,1) | 6' | 7,33(8,3 und 7,8) | 7,34(8,25 und 7,65) |
| 10 | 7,1 | 7,2 | 7' | 6,83(7,8) | 6,8(7,65) |
| 8 OH | 9,98 | 9,95 | 8'OH | 9,90 | 9,70 |
| 9 OH | 16,00 | 15,99 | 9'OH | 16,38 | 16,15 |

Lösungsmittel DMSO; J in Klammern ist in Hz angegeben

Tabelle 2

$^{13}C$ NMR von T 514 und T 514'

| C | T 514 | | T 514' | | C | T 514 | | T 514' | |
|---|---|---|---|---|---|---|---|---|---|
| 1 | 203,2 | a | 205,28 | f | 1' | 203,7 | a | 205,53 | f |
| 2 | 50,84 | | 50,83 | | 2' | 51,16 | | 51,02 | |
| 3 | 71,02 | b | 69,68 | g | 3' | 70,08 | b | 69,21 | g |
| 4 | 43,14 | | 42,41 | | 4' | 40,99 | | 40,64 | |
| 4a | 135,13 | | 138,60 | | 4'a | 135,13 | | 138,60 | |
| 5 | 118,59 | | 118,79 | | 5' | 117,01 | | 116,50 | |
| 6 | 135,16 | | 135,09 | | 6' | 132,63 | | 132,14 | |
| 7 | 119,93 | | 119,40 | | 7' | 111,159 | | 110,45 | |
| 8 | 155,38 | | 154,47 | | 8' | 158,51 | | 157,62 | |
| 8a | 112,98 | c | 112,10 | h | 8'a | 112,81 | c | 112,02 | h |
| 9 | 165,61 | | 163,98 | | 9' | 165,49 | | 163,63 | |
| 9a | 109,22 | d | 109,46 | i | 9'a | 109,74 | d | 109,98 | i |
| 10 | 118,50 | | 117,87 | | 10' | 125,26 | | 124,61 | |
| 10a | 139,34 | | 137,18 | | 10'a | 139,34 | | 137,18 | |
| $CH_3$ | 29,12 | e | 28,93 | j | $CH_3'$ | 29,22 | e | 29,02 | j |

Lösungsmittel DMSO; a,b,c,d,e,f,g,h,i,j an diesen Stellen kann die Zuordnung auch umgekehrt sein.

Beispiel 3

Gewinnung von T-510 der Formel:

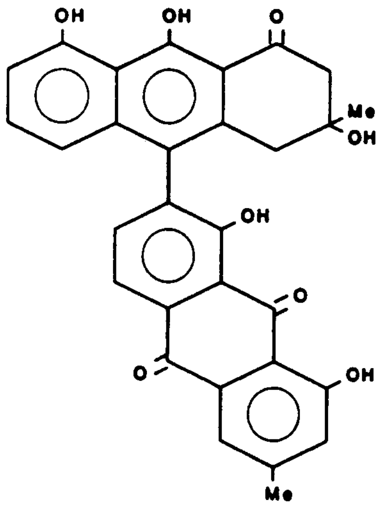

(T-510)

500 g getrocknete, zerkleinerte Früchte der Karwinskia affin humboldtiana werden nacheinander mit 3 l Petroläther (Siedebereich 60 - 70 ° C) und 3 l Chloroform extrahiert. Der Chloroformextrakt wird auf 100 ml konzentriert und das Produkt durch Zugabe von 300 ml n-Hexan gefällt. Das erhaltene Pulver wird durch Schichtchromatographie an Kieselgel mit Benzol:Aceton (3:1) als Lösungsmittel gereinigt. Die Fraktion mit dem höchsten $R_f$ wird an einer Kieselgelsäule mit Benzol:Aceton (40:1) als Laufmittel gereinigt. Die erhaltene Fraktion wird 24 h dem Licht ausgesetzt, filtriert und dann gereinigt durch Chromatographie an acetyliertem Polyamid mit $CHCl_3$/MeOH (30:1) und gefällt mit einer Mischung aus Benzol:Aceton. Ausbeute 60 mg.

Schmelzpunkt 136 - 138 ° C

UV : λ max. 423 (4.28)

314 (4.78)

264 (4.70)

226 (4.78)

IR ($cm^{-1}$) 3400, 1720, 1690, 1630, 1270, 750

$^1$H-NMR

| H | δ | | H | δ | |
|---|---|---|---|---|---|
| 2 | 7.14 | | 2' | 2.88 | |
| 3-Me | 2.5 | | 3'-$CH_3$ | 1.4 | |
| 4 | 7.67 | | 4' | 2.7 | (J=16 Hz) |
| | | | | 2.9 | (J=16 Hz) |
| 5 | 8.06 | (J=7.6) | 5' | 6.68 | (J=8.2Hz) |
| 6 | 7.66 | (J=7.6) | 6' | 7.4 | (J=8.3Hz) |
| | | | 7' | 6.88. | (J=8.3Hz) |
| 8-OH | 12.02 | | 8'-OH | 10.1 | |
| 1-OH | 12.37 | | 9'-OH | 16.04 | |

Beispiel 4

Gewinnung von T-496 der Formel:

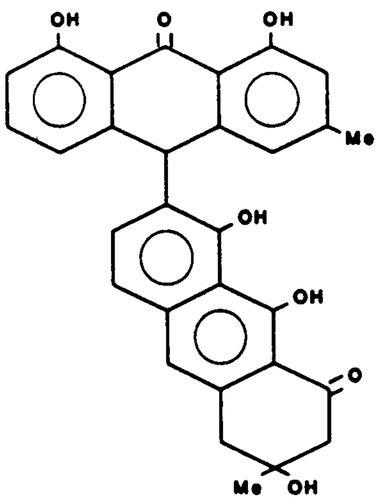

(T-496)

500 g getrocknete, zerkleinerte Früchte der Karwinskia humboldtiana werden nacheinander mit 3 l Petroläther (Siedebereich ungefähr 60 bis 70 °C) und 3 l Chloroform extrahiert. Der Chloroformextrakt wird auf 100 ml konzentriert und das Produkt durch 2-malige Zugabe von 300 ml n-Hexan gefällt. Das erhaltene gelbe Pulver wird an Silikagel G (Schichtdicke 0,5 mm) mit Benzol:Aceton (2 : 1) als Laufmittel fraktioniert. Die Fraktion mit dem höchsten $R_f$-Wert wird an einer Säule mit Kieselgel mit Benzol:Aceton (40 : 1) als Lösungsmitttel, chromatographiert. Das reine Produkt wird mit Benzol-Hexan gefällt. Ausbeute: 50 mg reines Pulver.

Schmelzpunkt 230 °C

UV: λ max 227 (4.66)

273 (4.71)

397 (4.15)

IR: (cm $^{-1}$) 3415, 1635, 1620, 1600

$^1$H-NMR

| H | δ | H | δ |
|---|---|---|---|
| 2 | 2.00 | 1'-OH | 12.20 |
| 3-Me | 1.40 | 2' | 6.60 |
| 4 | 3.05 | 3'-Me | 2.20 |
| 5 | 7.15 | 4' | 6.60 |
| 6 | 7.15 | 5' | 6.75 |
| | | 6' | 7.3 |
| 8-OH | 10.00 | 7' | 6.8 |
| 9-OH | 16.00 | | |
| 10 | 6.90 | 8'-OH | 12.30 |
| | | 10' | 6.10 |

Beispiel 5

Gewinnung von T ISO 514 der Formel:

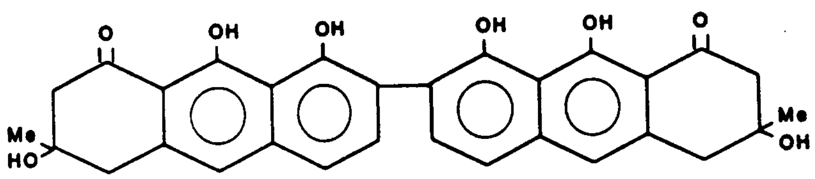

500 g getrocknete, zerkleinerte Früchte der Karwinskia umbelleta werden nacheinander mit 3 l Petroläther (Siedepunkt 60 - 70 °C) und 3 l Chloroform extrahiert. Der Chloroformextrakt wird auf 100 ml konzentriert und das Produkt durch Zugabe von 300 ml n-Hexan gefällt. Das erhaltene Pulver wird durch Schichtchromatographie an Kieselgel mit Benzol:Aceton (2:1) als Lösungsmittel, gereinigt. Die Fraktion mit dem niedrigsten $R_f$ wird einer Säulenchromatographie mit Kieselgel unterzogen. Das Produkt wird mit Benzol:Aceton (1:1) eluiert und dann an einer Sephadexsäule LH mit MeOH als Lösungsmittel gereinigt. Durch Fällung mit n-Hexan erhält man 30 mg reines Produkt.

Schmelzpunkt : 161 - 164 °C

UV : λ max. 422 (3.75)

270 (4.64)

220 (4.1 )

IR (cm $^{-1}$) 3400, 1630, 1600

$^{1}$H-NMR

| H | δ | |
|---|---|---|
| H- 2 | 2.7 | (J = 16) |
| | 3.0 | (J = 16) |
| 3-CH3 | 2.4 | |
| H- 4 | 3.5 | (J = 16) |
| | 3.10 | (J = 16) |
| H- 5 | 7.58 | (J = 8.4) |
| H- 6 | 7.3 | (J = 8.4) |
| H-10 | 7.14 | |
| 8 - OH | 9.9 | |
| 9 - OH | 16.05 | |

Beispiel 6

Gewinnung von T-544 der Formel:

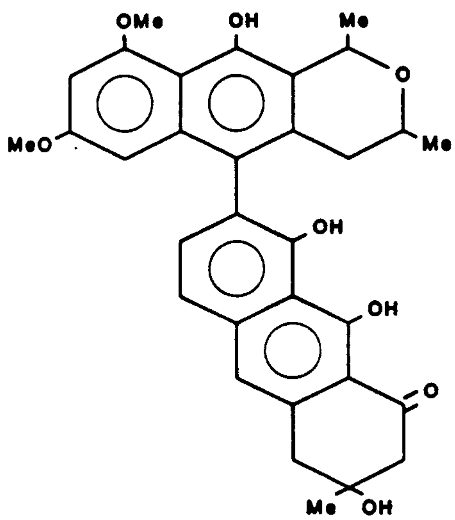

500 g getrocknete, zerkleinerte Früchte der Karwinskia humboldtiana werden nacheinander mit 3 l Petroläther (Siedebereich ungefähr 60 - 70 °C und 3 l Chloroform extrahiert. Der Chloroformextrakt wird auf 100 ml konzentriert und das Produkt durch zweimalige Zugabe von 300 ml n-Hexan gefällt. das erhaltene gelbe Pulver wird an Silikagel G (Schichtdicke 0,5 mm) mit Benzol;Aceton (2:1) als Laufmittel fraktioniert. Die Fraktion mit dem mittleren $R_f$-Wert wird durch Säulenchromatographie an Silikagel und Benzol/Aceton (20:1) als Laufmittel gereinigt. Nach erneuter Chromatographie und Fällung mit n-Hexan erhält man 200 mg reines Produkt.

Schmelzpunkt : 166 - 168 °C

UV (MeOH) λ max. 228 (4.67)

242 (4.77)

270 (4.63)

415 (4.00)

IR ($cm^{-1}$) 3390, 1625

## $^1$H-NMR

| H | | H | |
|---|---|---|---|
| | | 1' | 5.26 |
| 2 | 2.85 | 1' -Me | 1.69 |
| 3 Me | 1.46 | 3' | 3.7 |
| | | 3'-Me | 1.21 |
| 4 | 3.1 | 4' | 2.35 |
| 5 | 7.26 | 6' | 6.27 |
| 6 | 7.41 | 7'-O Me | 3.56 |
| 10 | 7.00 | 8' | 6.41 |
| | | 9'- O Me | 4.00 |
| 8-OH | 9.8 | | |
| 9-OH | 16.00 | 10' | 9.6 |

Beispiel 7

Cytotoxische Wirkung von T-514

Für den Test wurden Zellen menschlichen Ursprungs verwendet. Als hepatische Zellen wurden gutartige Chang-Leberzellen und als neoplastische Zellen drei Zellinien verwendet, nämlich Hepatom PLC/PRF/5, Hep3B mit dem Oberflächenantigen von Hepatitis B und Hep2B ohne Antigen.

Als Zellen pulmonalen Ursprungs wurden gutartige Lungenepithelzellen Wi 1003 und als neoplastische Zellen vier Zellinien verwendet, nämlich Schuppenkarzinom SK-mes-1, Adenokarzinom Calu, undifferenziertes bronchogenes Karzinom Cha-Go-K-1 und Kleinzellenkarzinom NCI-H 69.

Als Kolonzellen wurden gutartige Kolonepithelzellen CCD-33Co und als neoplastische Zellen die Kolonadenokarzinom-Zellinie LoVo.

Als Zellkulturmedium für alle untersuchten Zellinien kam eine Mischung aus Eagle's Basalmedium und Schaf-Fötusserum (9 + 1) zur Anwendung. Die Testsubstanzen (in Äthanol oder Wasser gelöst) wurden den Zellkulturen in den nachstehenden Konzentrationen (µg/ml) hinzugefügt.

| | |
|---|---|
| T-514 | 2.5, 5, 10, 20, 40, 80, 160,320 |
| Doxorubicin | 0.025, 0.05, 0.1, 0.2, 0.4, 0.8, 1.6, 3.2, 6.4 |
| 4-Epidoxorubicin | 0.025, 0.05, 0.1, 0.2, 0.4, 0.8, 1.6, 3.2, 6.4 |
| Vincristin | 0.002, 0.004, 0.008, 0.0156, 0.03125, 0.0625, 0.125, 0.25, 0.05, 1, 2, 4, 8, 16 |
| 5-Fluoruracil | 6.25, 12.5, 25, 50, 100, 200, 400, 800, 1600, 3200 |
| Mitomycin | 0.25, 0.5, 1, 2, 3, 4, 8, 16 |

Für die Kontrollversuche wurde das Lösungsmittel ohne die Testsubstanzen eingesetzt.

Die Auswertung nach 72-stündiger Inkubation erfolgte in üblicher Weise unter Bestimmung der Plattenhaftung, Morphologie und Proliferation der Zellen.

Die Ergebnisse sind als modifizierter therapeutischer Index $LD_{05\%}$ / $ED_{95\%}$ angegeben.

Die Werte wurden aus den entsprechenden Kurven extrapoliert. Der modifizierte therapeutische Index zeigt die Selektivität der Testsubstanzen auf, das heißt, ein positiver therapeutischer Index bedeutet, daß die neoplastischen Zellen empfindlicher auf die Testsubstanzen reagieren als gutartige Zellen, während dies bei einem negativen modifizierten therapeutischen Index genau umgekehrt ist. Die Ergebnisse sind in der Tabelle 3 zusammengestellt.

Tabelle 3

Modifizierter Therapeutischer Index

$$\frac{LD_{05\%}}{ED_{95\%}}$$

| | DOXO | EPI | MITO | VINC | FLUO | T-514 |
|---|---|---|---|---|---|---|
| $H_2$ | -1.877 | -1.119 | -1.869 | -13.636 | -2.588 | 14.257 |
| $H_3$ | -14.973 | -7.459 | -1.555 | -2035.818 | -2.214 | 8.565 |
| $H_4$ | -14.973 | -8.963 | -1.555 | -435.100 | -3.110 | 8.565 |
| IND. | 1.073 | 2.146 | -1.867 | -15.189 | -1.107 | 4.282 |
| ADE. | -1.867 | -1.071 | -1.555 | -15.189 | -1.107 | 4.282 |
| SCH. | -3.730 | -1.865 | -1.867 | -30.189 | -2.588 | 2.573 |
| KLE. | -3.106 | -1.553 | -1.555 | -15.189 | -6.620 | 8.565 |
| K.K. | -2.589 | -3.313 | 2.573 | -7.426 | 5.060 | 17.129 |

| | | CODE |
|---|---|---|
| $H_2$ | Hepatomazelle | PLC/PRF/5 |
| $H_3$ | Leberzellkarzinom | HEP 3B |
| $H_4$ | Leberzellkarzinom | HEP G2 |
| IND. | Bronchialkarzinom | Cha Go K-1 |
| ADE. | Lungenadenokarzinom | Calu-3 |
| SCH. | Lungenschuppenzellenkarzinom | SK-Mes-1 |
| KLE. | Lungenkleinzellenkarzinom | NCI-B69 |
| K.K. | Kolonadenokarzinom | LoVo |

| | | | |
|---|---|---|---|
| DOXO | DOXORUBICIN | VINC | VINCRISTIN |
| EPI | EPIDOXORUBICIN | FLUO | 5-FLUORURACIL |
| MITO | MITOMYCIN | | |

Die Ergebnisse zeigen, daß die Verbindung T-514 sehr selektiv wirkt, das heißt, eine größere Aktivität gegenüber malignen als gegenüber benignen Tumorzellen besitzt. Die erfindungsgemäßen Verbindungen sind daher brauchbare Cytostatika und Tumortherapeutika mit einem großen Sicherheitsabstand.

## Patentansprüche

**Patentansprüche für folgende Vertragsstaaten : AT, BE, CH, LI, DE, DK, FR, GB, IT, LU, NL, SE**

**1.** Pharmazeutisches Mittel, enthaltend wenigstens eine Verbindung der Formel 1:

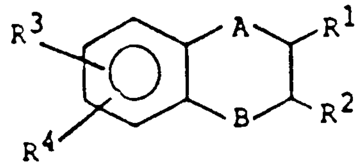

1

worin

| | |
|---|---|
| A | für C = O oder C-OH steht und |
| B | für CH-$R^5$ oder C-$R^5$ steht, wobei der die Gruppen A und B aufweisende Ring aromatisch ist, wenn A für C-OH und B für C-$R^5$ stehen, |
| $R^1$ und $R^2$ | , die gleich oder verschieden sein können, für eine $C_1$-$C_4$-Acylgruppe, $C_1$-$C_4$-Alkylgruppe, $C_1$-$C_4$-Alkoxygruppe steht oder $R^1$ und $R^2$ zusammen mit den Kohlenstoffatomen, an die sie gebunden sind, für einen Phenyl-, Cyclohexanon- oder Tetrahydropyranring stehen, der durch mindestens einen Rest substituiert sein kann, der ausgewählt ist unter einer $C_1$-$C_4$-Alkylgruppe, $C_1$-$C_4$-Alkoxygruppe, Hydroxygruppe, und $C_1$-$C_4$-Acyloxygruppe. |
| $R^3$ | für eine $C_1$-$C_4$-Alkylgruppe, $C_1$-$C_4$-Alkoxygruppe, $C_1$-$C_4$-Acyloxygruppe oder Hydroxygruppe steht, und |

einer der Reste $R^4$ und $R^5$ für ein Wasserstoffatom und der andere für eine Gruppe der Formel:

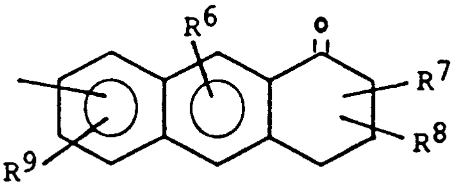

steht, worin $R^6$, $R^7$, $R^8$ und $R^9$ eine $C_1$-$C_4$-Alkylgruppe, $C_1$-$C_4$-Alkoxygruppe, $C_1$-$C_4$ Acyloxygruppe oder Hydroxygruppe bedeuten, oder eine tautomere Form, Stellungsisomer oder optisches Isomer davon, gegebenenfalls zusammen mit üblichen pharmazeutichen Trägern und/oder Hilfsstoffen.

**2.** Mittel nach Anspruch 1, enthaltend eine Verbindung der Formel

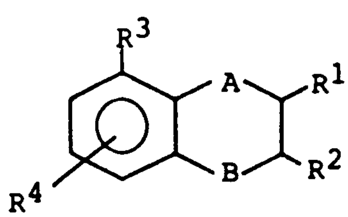

worin A, B, und $R^1$ bis $R^4$ die in Anspruch 1 angegebenen Bedeutungen besitzen.

3. Mittel nach Anspruch 1, enthaltend eine Verbindung einer der Formeln 1.1, 1.2 und 1.3:

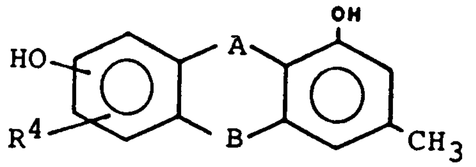

1.1

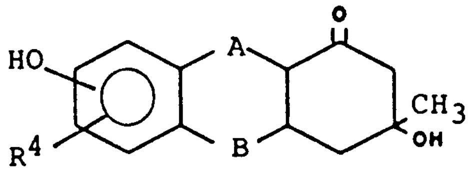

1.2

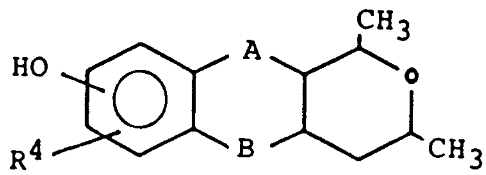

1.3

worin A, B und $R^4$ die in Anspruch 1 angegebenen Bedeutungen besitzen.

4. Mittel nach Anspruch 1 oder 2, wobei $R^1$ eine $C_1$-$C_4$-Acylgruppe, insbesondere eine Acetylgruppe, und $R^2$ eine $C_1$-$C_4$-Alkylgruppe, insbesondere eine Methylgruppe, bedeuten.

5. Mittel nach einem der vorhergehenden Ansprüche, wobei einer der Reste $R^4$ und $R^5$ für eine Gruppe der Formel:

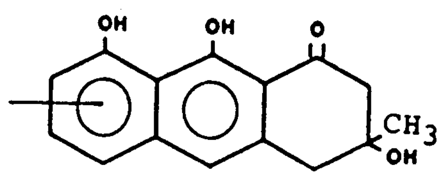

steht.

**6.** Mittel nach Anspruch 1, enthaltend eine Verbindung einer der Formeln I bis VI:

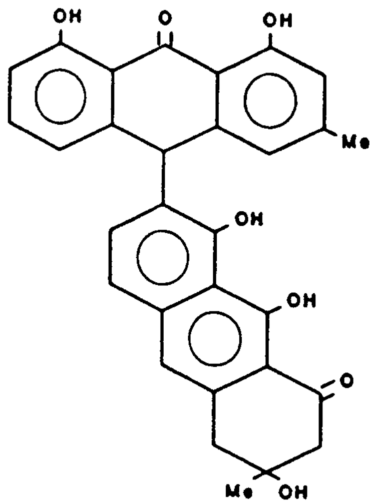

(I)

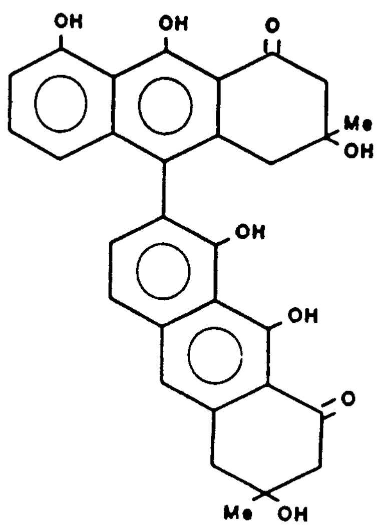

(II)

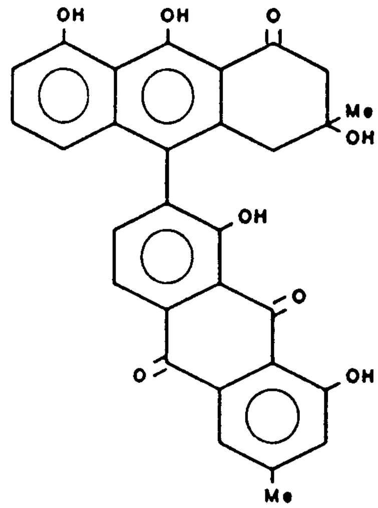

(III)

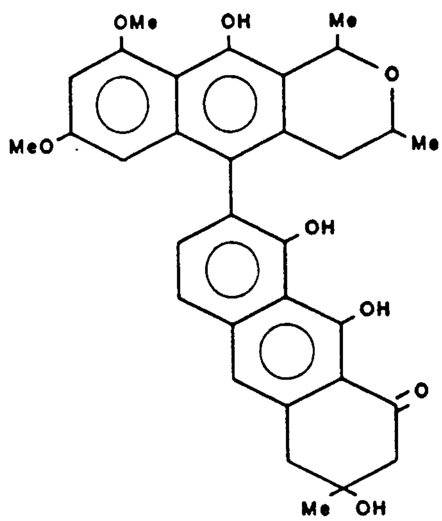

(IV)

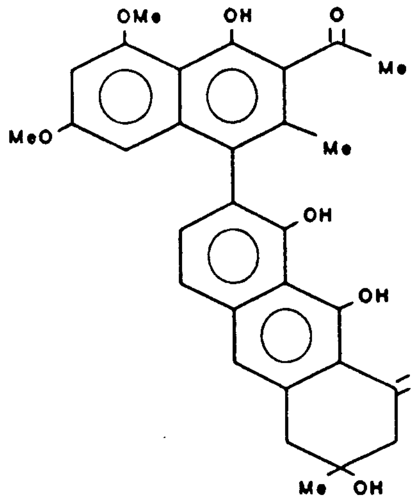

(V)

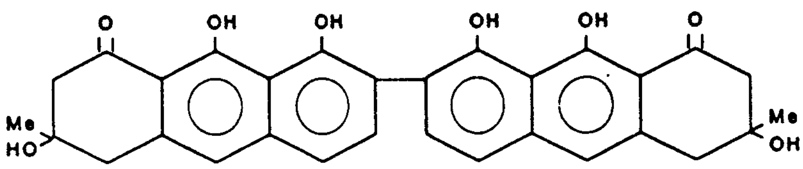

(VI)

**7.** Mittel nach Anspruch 5, enthaltend eine Verbindung der Formel II:

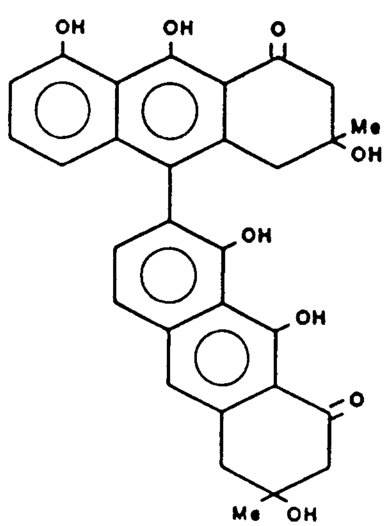

**8.** Verwendung wenigstens einer wie in einem der Ansprüche 1 bis 7 definierten Verbindung zur Herstellung eines cytostatischen oder antiviralen pharmazeutischen Mittels.

**9.** Verwendung nach Anspruch 8 zur Herstellung eines pharmazeutischen Mittels zur Tumorbehandlung, insbesondere zur Behandlung von Leber-, Lungen- und Kolontumoren.

**Patentansprüche für folgende Vertragsstaaten : ES, GR**

**1.** Verfahren zur Herstellung eines pharmazeutischen Mittels, enthaltend wenigstens eine Verbindung der Formel 1:

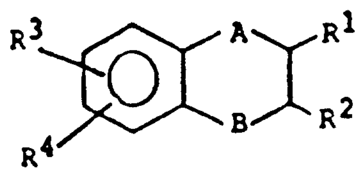

1

worin

| | |
|---|---|
| A | für C = O oder C-OH steht und |
| B | für CH-$R^5$ oder C-$R^5$ steht, wobei der die Gruppen A und B aufweisende Ring aromatisch ist, wenn A für C-OH und B für C-$R^5$ stehen, |
| $R^1$ und $R^2$ | , die gleich oder verschieden sein können, für eine $C_1$-$C_4$-Acylgruppe, $C_1$-$C_4$-Alkylgruppe, $C_1$-$C_4$-Alkoxygruppe steht oder $R^1$ und $R^2$ zusammen mit den Kohlenstoffatomen, an die sie gebunden sind, für einen Phenyl-, Cyclohexanon- oder Tetrahydropyranring stehen, der durch mindestens einen Rest substituiert sein kann, der ausgewählt ist unter einer $C_1$-$C_4$-Alkylgruppe, $C_1$-$C_4$-Alkoxygruppe, Hydroxygruppe, und $C_1$-$C_4$-Acyloxygruppe. |
| $R^3$ | für eine $C_1$-$C_4$-Alkylgruppe, $C_1$-$C_4$-Alkoxygruppe, $C_1$-$C_4$-Acyloxygruppe oder Hydroxygruppe steht, und |

einer der Reste $R^4$ und $R^5$ für ein Wasserstoffatom und der andere für eine Gruppe der Formel:

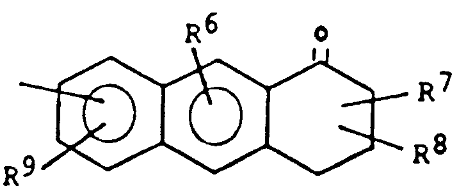

steht, worin $R^6$, $R^7$, $R^8$ und $R^9$ eine $C_1$-$C_4$-Alkylgruppe, $C_1$-$C_4$-Alkoxygruppe, $C_1$-$C_4$ Acyloxygruppe oder Hydroxyqruppe bedeuten,
oder einer tautomeren Form, eines Stellungsisomers oder optischen Isomers davon,
wobei man die Verbindung der Formel 1 in einer zur Verabreichung geeigneten Form bereitstellt, gegebenenfalls aufgenommen in üblichen pharmazeutischen Trägern und/oder Hilfsstoffen.

**2.** Verfahren nach Anspruch 1, wobei das Mittel eine Verbindung der Formel

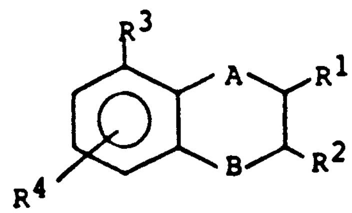

worin A, B, und $R^1$ bis $R^4$ die in Anspruch 1 angegebenen Bedeutungen besitzen, enthält.

**3.** Verfahren nach Anspruch 1, wobei das Mittel eine Verbindung einer der Formeln 1.1, 1.2 und 1.3:

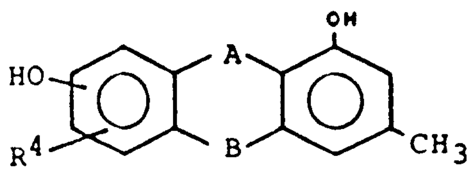

1.1

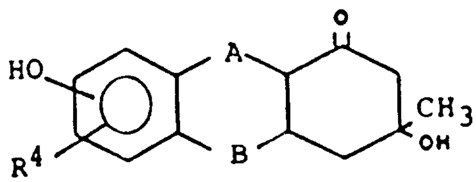

1.2

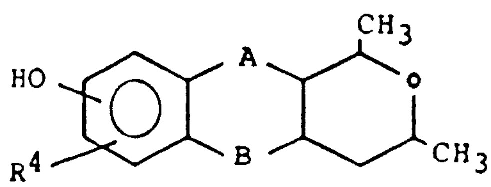

1.3

worin A, B und $R^4$ die in Anspruch 1 angegebenen Bedeutungen besitzen, enthält.

**4.** Verfahren nach Anspruch 1 oder 2, wobei $R^1$ eine $C_1$-$C_4$-Acylgruppe, insbesondere eine Acetylgruppe, und $R^2$ eine $C_1$-$C_4$-Alkylgruppe, insbesondere eine Methylgruppe, bedeuten.

**5.** Verfahren nach einem der vorhergehenden Ansprüche, wobei einer der Reste $R^4$ und $R^5$ für eine Gruppe der Formel:

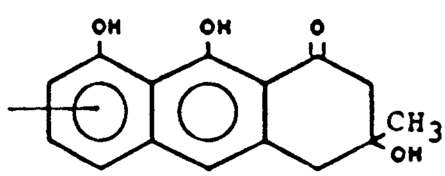

steht.

**6.** Verfahren nach Anspruch 1, wobei das Mittel eine Verbindung einer der Formeln I bis VI enthält:

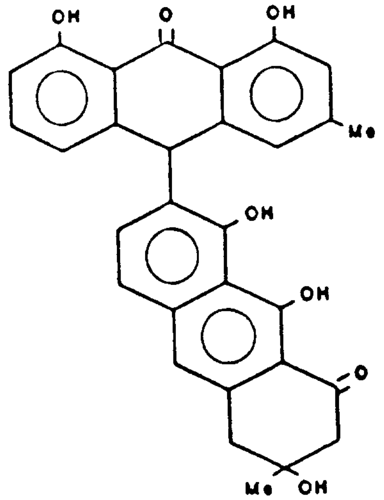

(I)

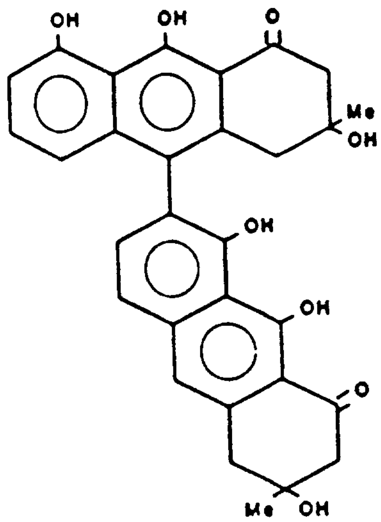

(II)

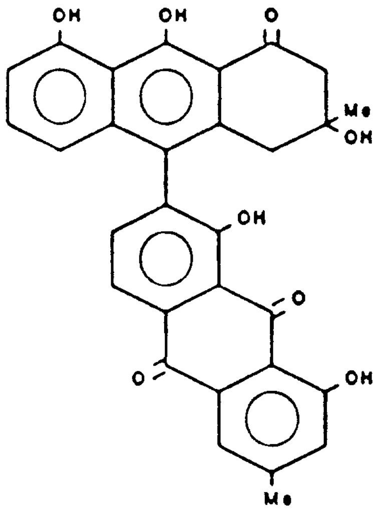

(III)

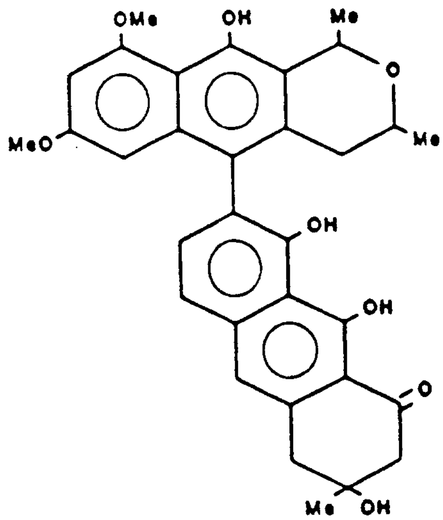

(IV)

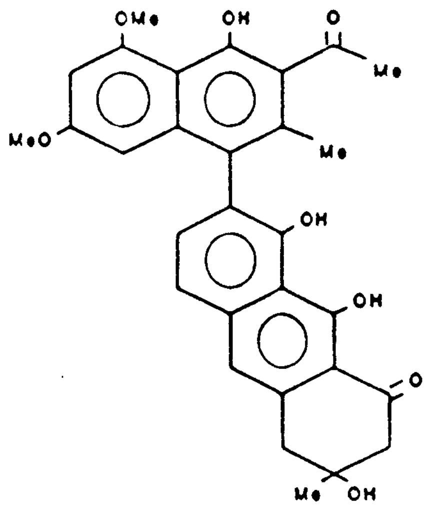

(V)

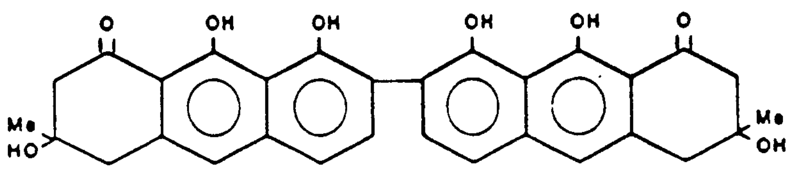

(VI)

7. Verfahren nach Anspruch 5, wobei das Mittel eine Verbindung der Formel II:

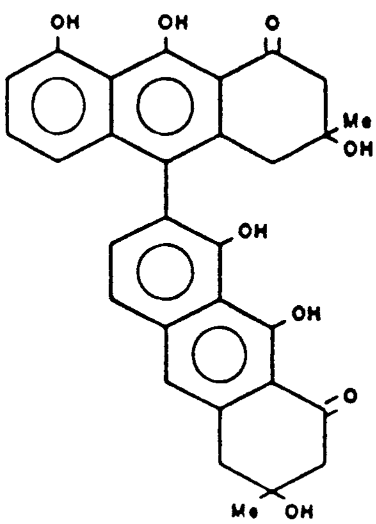

enthält.

8. Verwendung wenigstens einer wie in einem der Ansprüche 1 bis 7 definierten Verbindung zur Herstellung eines cytostatischen oder antiviralen pharmazeutischen Mittels.

9. Verwendung nach Anspruch 8 zur Herstellung eines pharmazeutischen Mittels zur Tumorbehandlung, insbesondere zur Behandlung von Leber-, Lungen- und Kolontumoren.

**Claims**
**Claims for the following Contracting States : AT, BE, CH, LI, DE, DK, FR, GB, IT, LU, NL, SE**

1. Pharmaceutical composition containing at least one compound of formula 1:

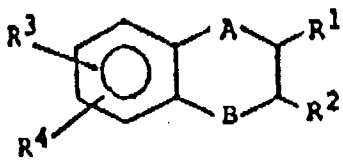

1

wherein

| | |
|---|---|
| A | denotes C = O or C-OH and |
| B | denotes CH-$R^5$ or C-$R^5$, wherein the ring containing the groups A and B is aromatic if A denotes C-OH and B denotes C-$R^5$, |
| $R^1$ and $R^2$ | , which may be identical or different, represent a $C_{1-4}$-acyl group, $C_{1-4}$-alkyl group or $C_{1-4}$-alkoxy group or $R^1$ and $R^2$ together with the carbon atoms to which they are bound represent a phenyl, cyclohexanone or tetrahydropyran ring which may be substituted by at least one group selected from among a $C_{1-4}$-alkyl, $C_{1-4}$-alkoxy, hydroxy or $C_{1-4}$-acyloxy group, |
| $R^3$ | denotes a $C_{1-4}$-alkyl, $C_{1-4}$-alkoxy, $C_{1-4}$-acyloxy or hydroxy group, and |

one of the groups $R^4$ and $R^5$ denotes a hydrogen atom and the other denotes a group of formula:

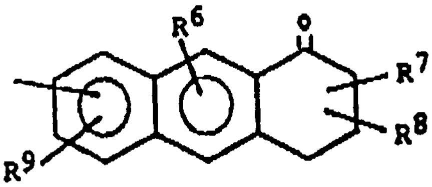

wherein $R^6$, $R^7$, $R^8$ and $R^9$ denote a $C_{1-4}$-alkyl, $C_{1-4}$-alkoxy, $C_{1-4}$-acyloxy or hydroxy group,
or a tautomeric form, position isomer or optical isomer thereof, optionally together with conventional pharmaceutical carriers and/or excipients.

2. Agent according to claim 1, containing a compound of formula

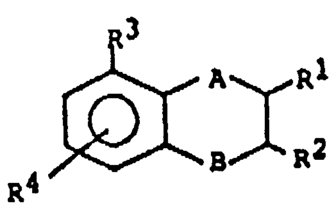

wherein A, B and $R^1$ to $R^4$ have the meanings given in claim 1.

3. Composition according to claim 1, containing a compound according to one of formulae 1.1, 1.2 and 1.3:

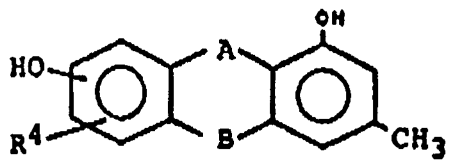

1.1

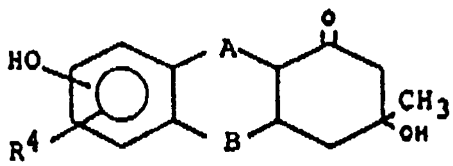

1.2

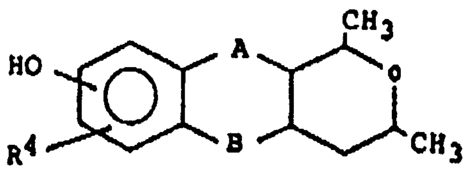

1.3

wherein A, B and $R^4$ have the meanings given in claim 1.

**4.** Composition according to claim 1 or 2, wherein $R^1$ denotes a $C_{1-4}$-acyl group, particularly an acetyl group, and $R^2$ denotes a $C_{1-4}$-alkyl group, particularly a methyl group.

**5.** Composition according to one of the preceding claims, wherein one of the groups $R^4$ and $R^5$ denotes a group of the formula:

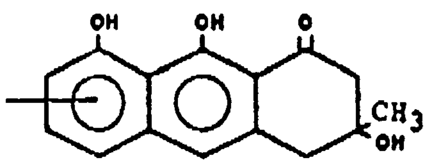

**6.** Composition according to claim 1, containing a compound according to one of formula I to VI:

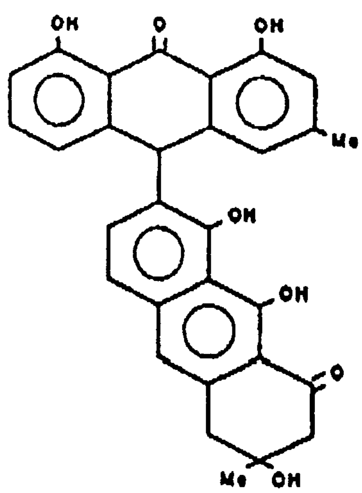

(I)

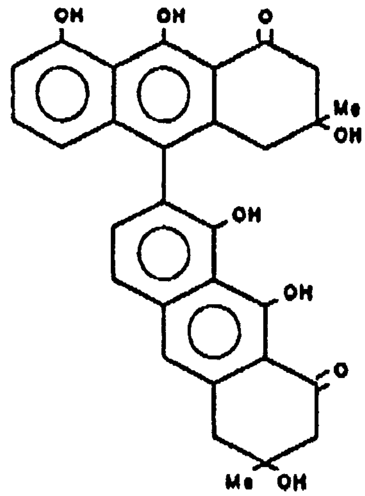

(II)

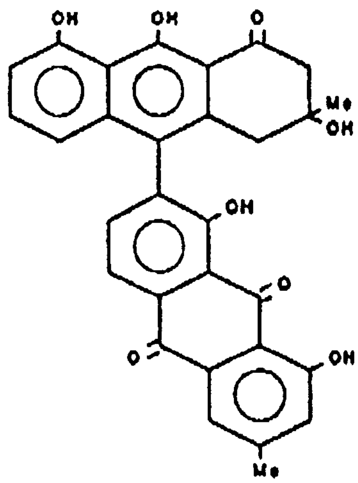

(III)

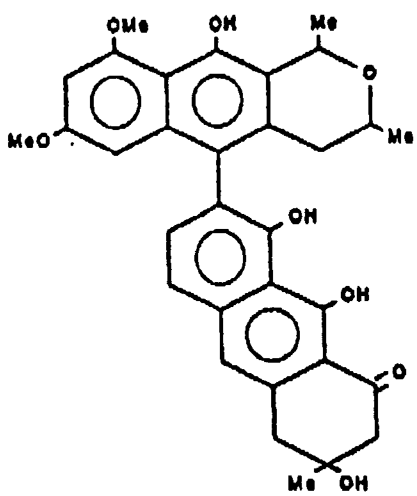

(IV)

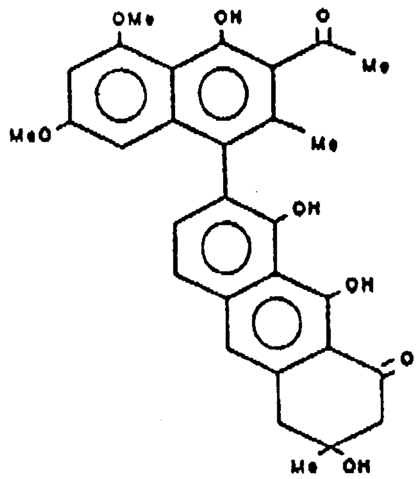

(V)

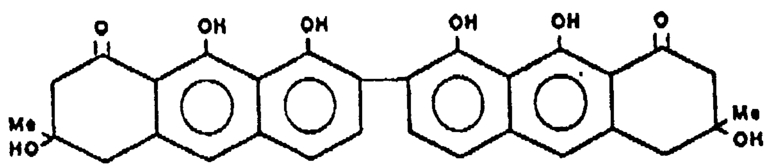

(VI)

**7.** Composition according to claim 5, containing a compound of formula II:

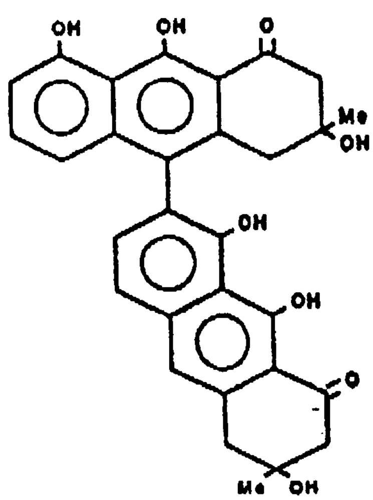

**8.** Use of at least one compound as defined in one of claims 1 to 7 for preparing a cytostatic or antiviral pharmaceutical composition.

9. Use according to claim 8 for preparing a pharmaceutical composition for the treatment of tumours, particularly for the treatment of tumours of the liver, lung and colon.

**Claims for the following Contracting States : ES, GR**

1. Process for preparing a pharmaceutical composition containing at least one compound of formula 1:

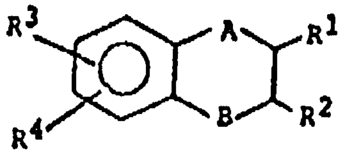

1

wherein

| | |
|---|---|
| A | denotes C = O or C-OH and |
| B | denotes CH-$R^5$ or C-$R^5$, wherein the ring containing the groups A and B is aromatic if A denotes C-OH and B denotes C-$R^5$, |
| $R^1$ and $R^2$ | , which may be identical or different, represent a $C_{1-4}$-acyl group, $C_{1-4}$-alkyl group or $C_{1-4}$-alkoxy group or $R^1$ and $R^2$ together with the carbon atoms to which they are bound represent a phenyl, cyclohexanone or tetrahydropyran ring which may be substituted by at least one group selected from among a $C_{1-4}$-alkyl, $C_{1-4}$-alkoxy, hydroxy or $C_{1-4}$-acyloxy group, |
| $R^3$ | denotes a $C_{1-4}$-alkyl, $C_{1-4}$-alkoxy, $C_{1-4}$-acyloxy or hydroxy group, and |

one of the groups $R^4$ and $R^5$ denotes a hydrogen atom and the other denotes a group of formula:

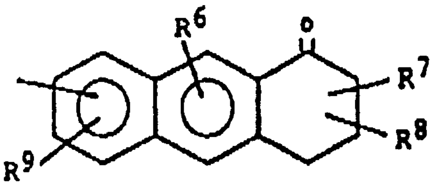

wherein $R^6$, $R^7$, $R^8$ and $R^9$ denote a $C_{1-4}$-alkyl, $C_{1-4}$-alkoxy, $C_{1-4}$-acyloxy or hydroxy group,
or a tautomeric form, position isomer or optical isomer thereof,
wherein the compound of formula 1 is prepared in a form suitable for administration, optionally incorporated in conventional pharmaceutical carriers and/or excipients.

2. Process according to claim 1, wherein the composition contains a compound of formula

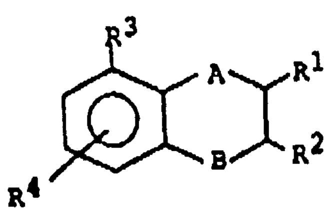

wherein A, B and $R^1$ to $R^4$ have the meanings given in claim 1.

3. Process according to claim 1, wherein the composition contains a compound of one of the formulae 1.1, 1.2 and 1.3:

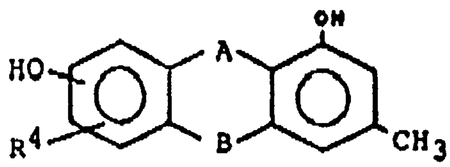

1.1

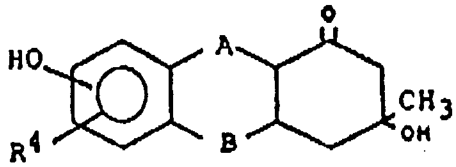

1.2

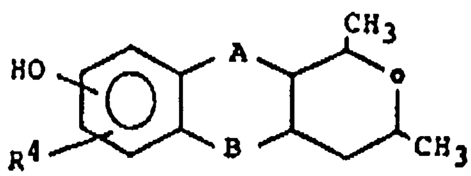

1.3

wherein A, B and $R^4$ have the meanings given in claim 1.

4. Process according to claim 1 or 2, wherein $R^1$ denotes a $C_{1-4}$-acyl group, particularly an acetyl group, and $R^2$ denotes a $C_{1-4}$-alkyl group, particularly a methyl group.

5. Process according to one of the preceding claims, wherein one of the groups $R^4$ and $R^5$ denotes a group of formula:

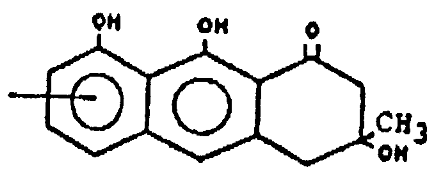

6. Process according to claim 1, wherein the composition contains a compound of one of the formulae I to VI:

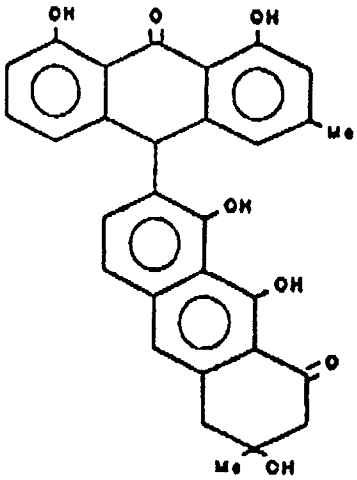

(I)

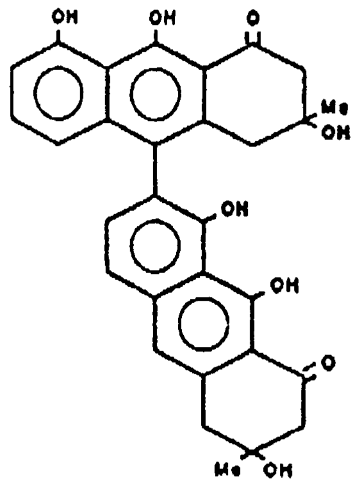

(II)

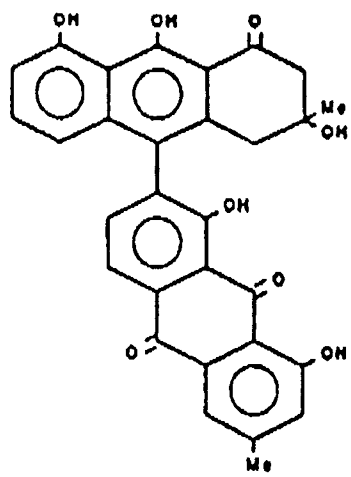

(III)

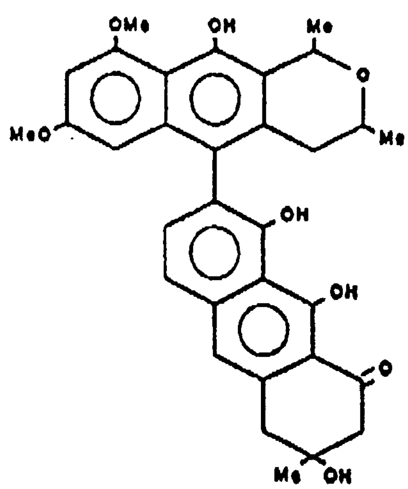

(IV)

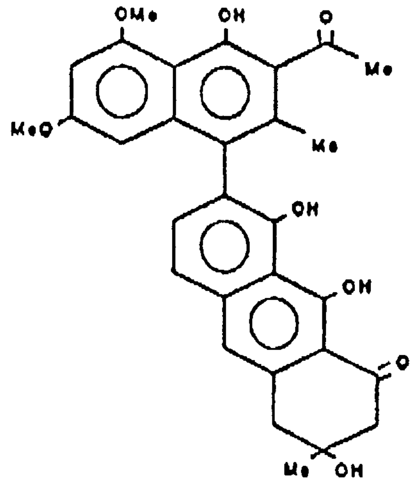

(V)

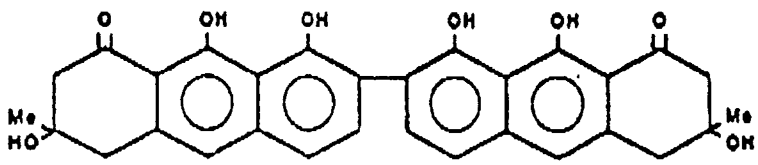

(VI)

7. Process according to claim 5, wherein the composition contains a compound of formula II:

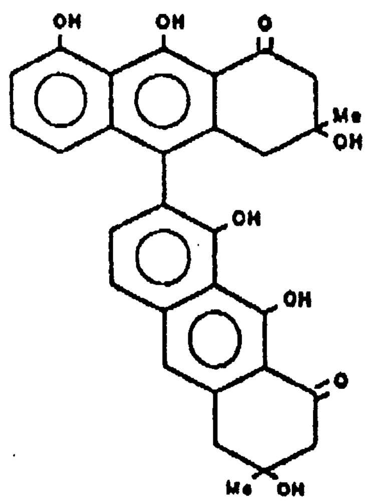

8. Use of at least one compound as defined in one of claims 1 to 7 for preparing a cytostatic or antiviral pharmaceutical composition.

**9.** Use according to claim 8 for preparing a pharmaceutical composition for the treatment of tumours, particularly for the treatment of tumours of the liver, lung and colon.

**Revendications**

**Revendications pour les Etats contractants suivants : AT, BE, CH, LI, DE, DK, FR, GB, IT, LU, NL, SE**

**1.** Composition pharmaceutique contenant au moins un composé de formule 1 :

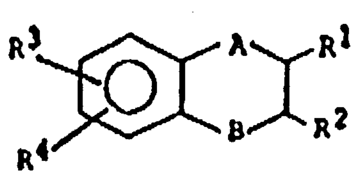

1

dans laquelle

| | |
|---|---|
| A | représente C = O ou C-OH et |
| B | représente CH-$R^5$ ou C-$R^5$, le cycle contenant les groupes A et B étant aromatique lorsque A est C-OH et B est C-$R^5$, |
| $R^1$ et $R^2$ | qui peuvent être identiques ou différents, représentent un radical acyle on $C_1$-$C_4$, un radical alkyle en $C_1$-$C_4$, un radical alcoxy on $C_1$-$C_4$, ou bien $R^1$ et $R^2$ ensemble, avec les atomes de carbone auxquels ils sont liés, représentent un cycle phényle, un cycle cyclohexanone ou un cycle tétrahydropyrane, substitué par au moins un radical choisi parmi les radicaux alkyle en $C_1$-$C_4$, alcoxy an $C_1$-$C_4$, hydroxy et acyloxy en $C_1$-$C_4$, |
| $R^3$ | représente un radical alkyle on $C_1$-$C_4$, un radical alcoxy en $C_1$-$C_4$, un radical acyloxy en $C_1$-$C_4$ ou un radical hydroxy, et |

l'un des radicaux $R^4$ ou $R^5$ représente un atome d'hydrogène alors que l'autre représente un radical de formule :

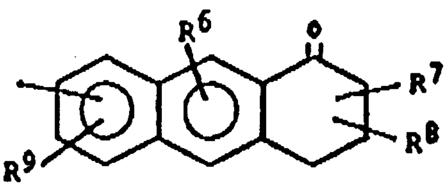

dans laquelle $R^6$, $R^7$, $R^8$ et $R^9$ représentent un radical alkyle an $C_1$-$C_4$, alcoxy en $C_1$-$C_4$, acyloxy en $C_1$-$C_4$ ou hydroxy,

ou bien une forme tautomère, un isomère de position ou un isomère optique de cette composition, éventuellement mélangée à des supports pharmaceutiques usuels et/ou à des additifs.

**2.** Composition selon la revendication 1, contenant un composé de formule :

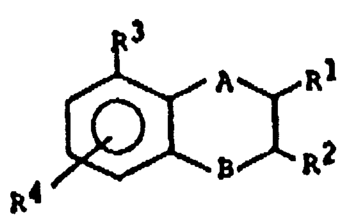

dans laquelle A, B et $R^1$ à $R^4$ ont les significations qui leur sont attribuées dans la revendication 1.

**3.** Composition selon la revendication 1, contenant un composé d'une des formules 1.1, 1.2 et 1.3 :

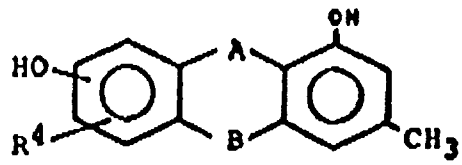

1.1

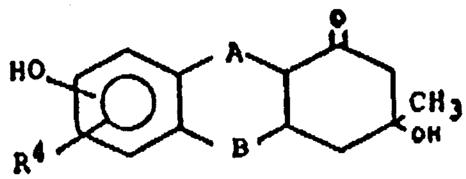

1.2

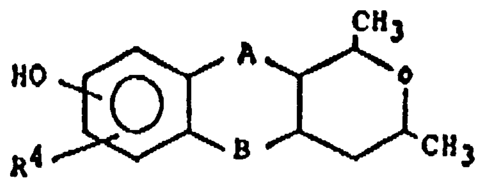

1.3

dans lesquelles A, B et $R^4$ ont les significations qui leur sont attribuées dans la revendication 1.

**4.** Composition selon l'une des revendications 1 ou 2, dans laquelle $R^1$ représente un radical acyle en $C_1$-$C_4$,en particulier un radical acétyle, et $R^2$ représente un radical alkyle en $C_1$-$C_4$, en particulier un radical méthyle.

**5.** Composition selon l'une des revendications précédentes, dans laquelle un des radicaux $R^4$ et $R^5$ représente un radical de formule :

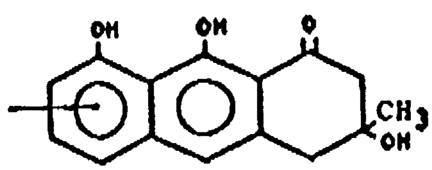

6. Composition selon la revendication 1, contenant un composé d'une des formules I à VI :

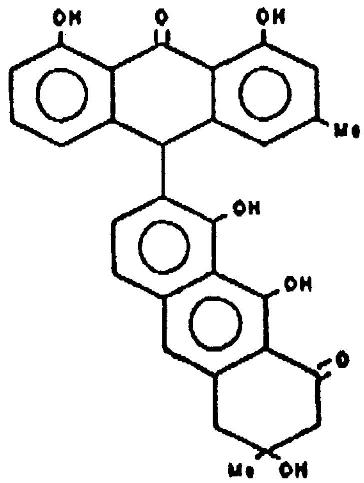

(I)

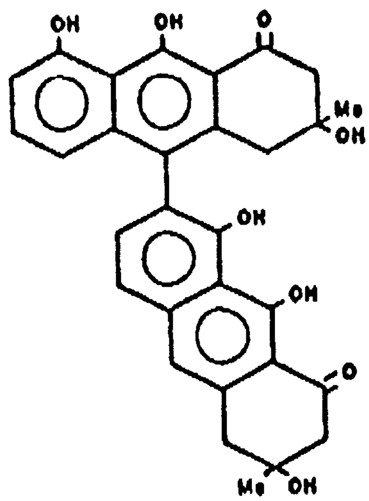

(II)

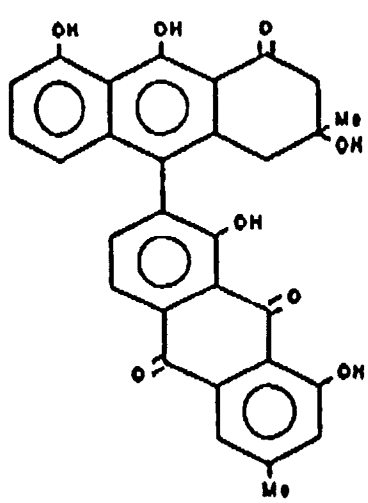

(III)

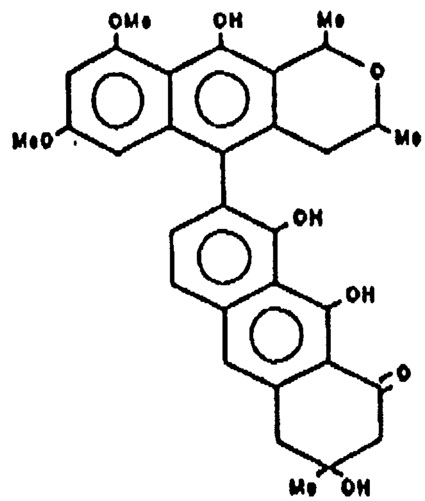

(IV)

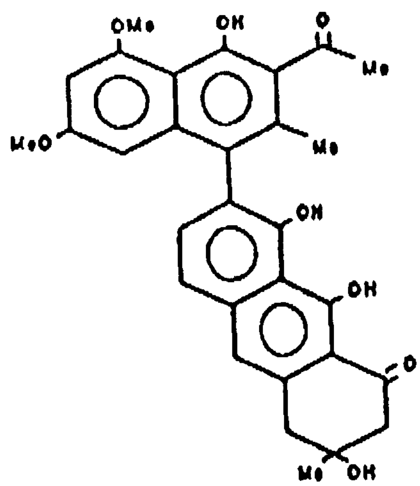

(V)

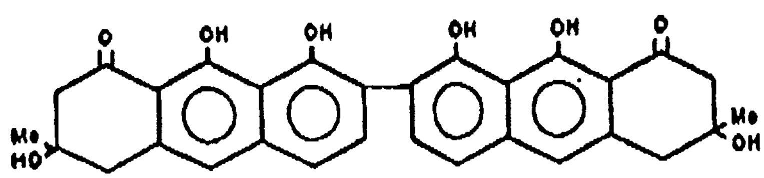

(VI)

**7.** Composition selon la revendication 5, contenant un composé de formule II :

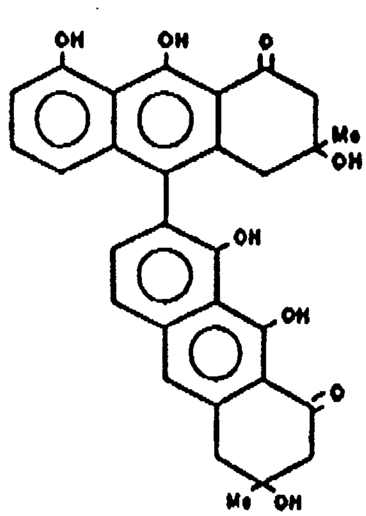

**8.** Utilisation d'au moins un des composés définis dans l'une des revendications 1 à 7, pour la préparation d'une composition pharmaceutique cytostatique ou antivirale.

**9.** Utilisation selon la revendication 8, pour la préparation d'une composition pharmaceutique destinée au traitement des tumeurs, on particulier au traitement de tumeurs du foie, des poumons et du colon.

**Revendications pour les Etats contractants suivants : ES, GR**

**1.** Procédé de préparation d'une composition pharmaceutique contenant au moins un composé de formule 1 :

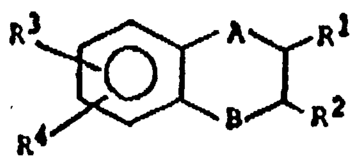

1

dans laquelle

| | |
|---|---|
| A | représente C = O ou C-OH et |
| B | représente CH-$R^5$ ou C-$R^5$, le cycle contenant les groupes A et B étant aromatique lorsque A est C-OH et B est C-$R^5$, |
| $R^1$ et $R^2$ | qui peuvent être identiques ou différents, représentent un radical acyle en $C_1$-$C_4$, un radical alkyle en $C_1$-$C_4$, un radical alcoxy en $C_1$-$C_4$, ou bien $R^1$ et $R^2$ ensemble, avec les atomes de carbone auxquels ils sont liés, représentent un cycle phényle, un cycle cyclohexanone ou un cycle tétrahydropyrane, substitué par au moins un radical choisi parmi les radicaux alkyle en $C_1$-$C_4$, alcoxy en $C_1$-$C_4$, hydroxy et acyloxy on $C_1$-$C_4$, |
| $R^3$ | représente un radical alkyle on $C_1$-$C_4$, un radical alcoxy en $C_1$-$C_4$, un radical acyloxy en $C_1$-$C_4$ ou un radical hydroxy, et |

l'un des radicaux $R^4$ ou $R^5$ représente un atome d'hydrogène alors que l'autre représente un radical de formule :

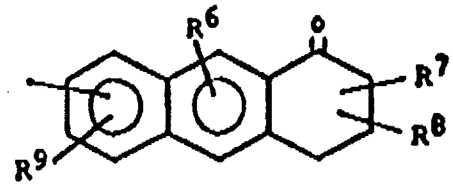

dans laquelle $R^6$, $R^7$, $R^8$ et $R^9$ représentent un radical alkyle en $C_1$-$C_4$, alcoxy on $C_1$-$C_4$, acyloxy en $C_1$-$C_4$ ou hydroxy,
ou bien une forme tautomère, un isomère de position ou un isomère optique do cette composition, selon lequel on met le composé de formule 1 sous une forme convenant pour son administration, éventuellement en mélange avec les supports pharmaceutiques usuels et/ou des additifs.

**2.** Procédé selon la revendication 1, dans lequel la composition contient un composé de formule :

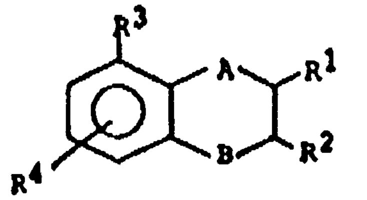

dans laquelle A, B et $R^1$ à $R^4$ ont les significations qui leur sont attribuées dans la revendication 1.

**3.** Procédé selon la revendication 1, dans lequel la composition contient un composé d'une des formules 1.1, 1.2 et 1.3 :

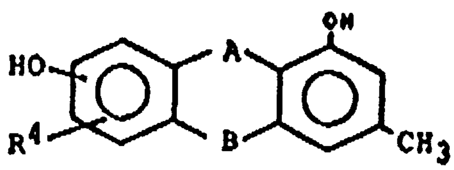

1.1

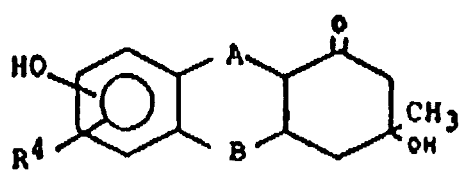

1.2

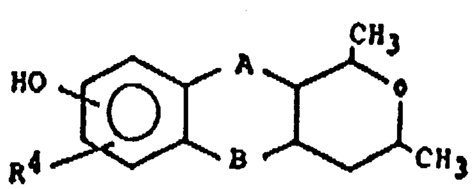

1.3

dans lesquelles A, B et $R^4$ ont les significations qui leur sont attribuées dans la revendication 1.

**4.** Procédé selon l'une des revendications 1 ou 2, dans lequel $R^1$ représente un radical acyle on $C_1$-$C_4$, en particulier un radical acétyle, et $R^2$ représente un radical alkyle en $C_1$-$C_4$, en particulier un radical méthyle.

**5.** Procédé selon l'une des revendications précédentes, dans lequel un des radicaux $R^4$ et $R^5$ représente un radical de formule :

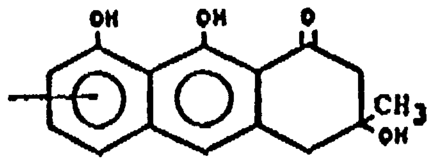

6. Procédé selon la revendication 1, dans lequel la composition contient un composé d'une des formules I à VI :

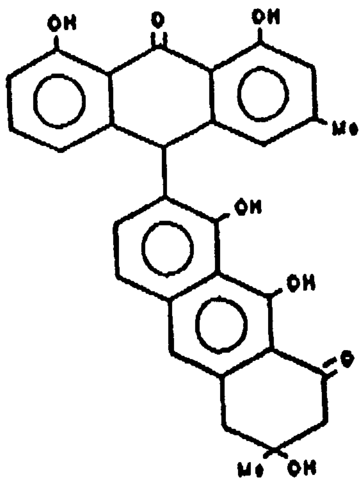

(I)

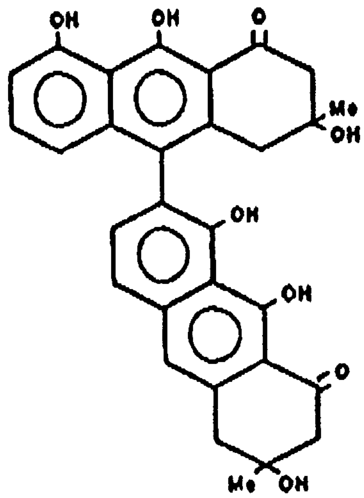

(II)

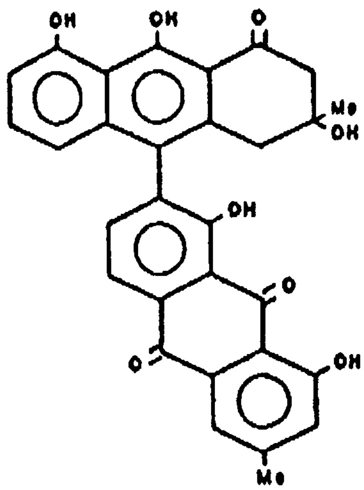

(III)

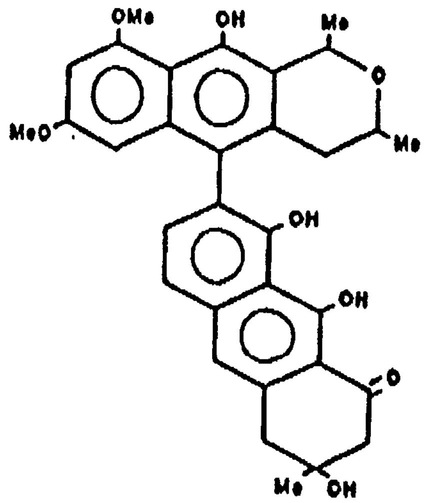

(IV)

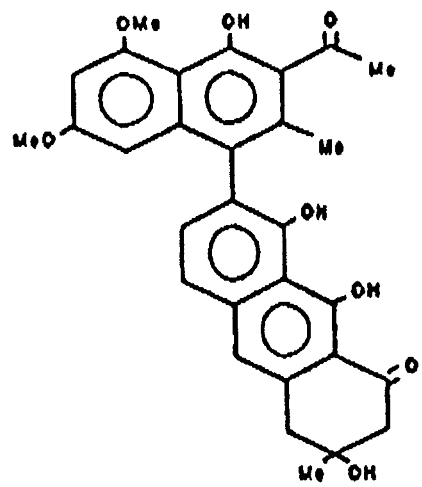

(V)

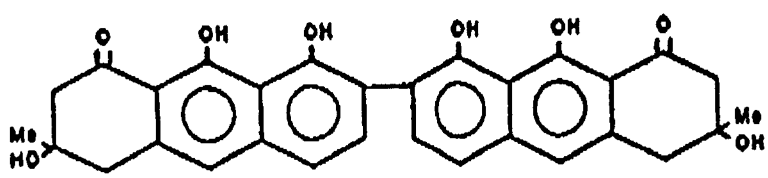

(VI)

**7.** Procédé selon la revendication 5, dans lequel la composition contient un composé de formule II :

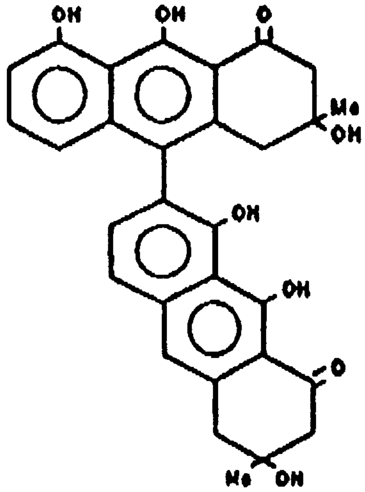

**8.** Utilisation d'au moins un des composés définis dans l'une des revendications 1 à 7, pour la préparation d'une composition pharmaceutique cytostatique ou antivirale.

**9.** Utilisation selon la revendication 8, pour la préparation d'une composition pharmaceutique destinée au traitement des tumeurs, en particulier au traitement de tumeurs du foie, des poumons et du colon.